# EUROPÄISCHE PATENTSCHRIFT

(11) **EP 0 724 564 B1**
(45) Veröffentlichungstag und Bekanntmachung des Hinweises auf die Patenterteilung: **22.04.1998**
(21) Anmeldenummer: 94929533.1
(22) Anmeldetag: 12.10.1994
(51) Int. Cl.: C07C 233/18, C07C 233/20, C11D 1/52

(54) **VERWENDUNG VON ENDGRUPPENVERSCHLOSSENEN FETTSÄUREAMIDALKOXYLATEN**
USE OF TERMINAL GROUP CAPPED FATTY ACID AMIDE ALKOXYLATES
UTILISATION D'AMIDE-ALKOXYLATES D'ACIDES GRAS BLOQUES PAR DES GROUPES TERMINAUX

(30) Priorität: 22.10.1993 DE 4336247
(43) Veröffentlichungstag der Anmeldung: 07.08.1996
(73) Patentinhaber: BASF AKTIENGESELLSCHAFT, 67056 Ludwigshafen (DE)
(72) Erfinder: STRECKER, Beate, D-67059 Ludwigshafen (DE); OETTER, Günter, D-67227 Frankenthal (DE); OFTRING, Alfred, D-67098 Bad Dürkheim (DE); PERNER, Johannes, D-67434 Neustadt (DE); BAUR, Richard, D-67112 Mutterstadt (DE); SCHWENDEMANN, Volker, D-67434 Neustadt (DE); AUS DEM KAHMEN, Martin, D-67071 Ludwigshafen (DE); REIF, Wolfgang, D-67227 Frankenthal (DE)
(86) Internationale Anmeldenummer: EP9403354
(87) Internationale Veröffentlichungsnummer: WO9511225

(56) Entgegenhaltungen:
- EP-A- 0 161 537
- EP-A- 0 564 402
- CH-A- 481 633
- US-A- 3 180 786

## Beschreibung

Die vorliegende Erfindung betrifft die Verwendung von endgruppenverschlossenen Fettsäureamidalkoxylaten der allgemeinen Formel I

R¹-CO-NH-(CH₂)ₙ-O-(AO)ₓ-R² (I)

in der
- R¹: einen C₅- bis C₂₁-Alkyl- oder -Alkenylrest bezeichnet,
- R²: eine C₁- bis C₄-Alkylgruppe bedeutet,
- A: für C₂- bis C₄-Alkylen steht,
- n: die Zahl 2 oder 3 bezeichnet und
- x: einen Wert von 2 bis 6 hat,
als nichtionische Tenside oder Emulgatoren in Wasch-, Reinigungs- oder Körperpflegemitteln, als oberflächenaktive Substanzen für industrielle Anwendungszwecke in der Galvanotechnik, in der photographischen Industrie, bei der Erdölförderung, in der pharmazeutischen Industrie, bei der Pflanzenernährung und in Pflanzenschutzformulierungen sowie als oberflächenaktive Substanzen bei der Herstellung von Emulsionen, Pigment- und Kunststoffdispersionen.

Weiterhin betrifft die Erfindung Wasch-, Reinigungs- und Körperpflegemittel, die die Verbindungen I enthalten. Da ein Teil der Verbindungen I neue Stoffe darstellt, betrifft die Erfindung auch diese neuen Stoffe. Gegenstand der Erfindung ist weiterhin ein Verfahren zur Herstellung dieser neuen Stoffe.

Wasch- und Reinigungsprozesse in Industrie, Gewerbebetrieben und Haushalt verlangen heutzutage immer mehr nach oberflächenaktiven Substanzen, welche sich vor allem durch gute Alkalistabilität, große Oberflächenspannungserniedrigung und gutes Netz- und Schäumvermögen auszeichnen, bei maschinell ablaufenden Reinigungsprozessen sind besonders Schaumarmut und effektive Schaumdämpfung gefragt. Weiterhin sollen diese oberflächenaktiven Substanzen biologisch weitgehend abbaubar sein und kein gesundheitsgefährdendes Potential in sich bergen.

In der DE-A 24 23 893 (1) wird ein Enteisungs- und Vereisungsschutzmittel für Flugzeuge beschrieben, welches einen Zusatz einer wasserdispergierbaren Verbindung der Formel α

R-X-(CH₂CH₂O)ₙ-Y (α)

in der R u.a. einen gegebenenfalls verzweigten C₁₂- bis C₂₄-Alkylrest, X u.a. die Gruppe -CO-NH-, n eine Zahl von 1 bis 30 und Y u.a. eine C₁- bis C₂₀-Alkylgruppe bezeichnet, enthält. Ein weiterer möglicher Zusatz für dieses Mittel ist eine wasserunlösliche Verbindung der Formel β

R⁴-A-(CHR¹CH₂O)ₘ-B (β)

in der R⁴ einen gegebenenfalls verzweigten und gegebenenfalls eine Hydroxylgruppe enthaltenden C₈- bis C₂₄-Alkylrest, A u.a. die Gruppe -CO-NH-, R¹ Wasserstoff oder Methyl, m eine Zahl von 1 bis 3 und B u.a. einen C₁- bis C₅-Alkylrest bedeutet.

Aus der US-A 3 746 644 (2) sind Ölsäurederivate der Formel

C₁₇H₃₄-CO-NH-(CH₂)₃-O-CH₂CH₂-O-CH₂CH₃

und

C₁₇H₃₄-CO-NH-(CH₂)₃-O-CH₂CH₂-O-CH₂CH₂-O-CH₂CH₃

bekannt. Diese Verbindungen eignen sich als Schmiermittel, die hohen Drücken standhalten und basenstabil sind.

Die EP-B 211 493 (3) betrifft eine flüssige Reinigungsmittelzusammensetzung, welche eine Mischung aus einem ionischen Tensid und einem Polyalkoxy-nichtionischen Tensid der allgemeinen Formel RVEW umfaßt. Dabei steht R für einen (ar)aliphatischen Kohlenwasserstoffanteil, V u.a. für die Gruppe -CO-NH-, E für Polyethoxy und/oder Polypropoxy (über den Alkoxylierungsgrad wird keine allgemeine Aussage gemacht, in den Beispielen liegt er bei 14 oder 20) und W für eine nichtionische Endgruppe, z.B. OH oder CH₃.

Die genannten Fettsäureamidalkoxylate des Standes der Technik erweisen sich aber in ihren wasch- und reinigungstechnischen Eigenschaften immer noch als verbesserungsbedürftig. Aufgabe der vorliegenden Erfindung war es daher, Tenside bzw. Emulgatoren für die Anwendung in Wasch-, Reinigungs- und Körperpflegemitteln mit verbessertem Eigenschaftsprofil bereitzustellen.

Demgemäß wurde die diesbezügliche Verwendung der eingangs definierten endgruppenverschlossenen Fettsäureamidalkoxylate I gefunden.

Der Rest R¹ bezeichnet C₅- bis C₂₁-Alkyl oder -Alkenyl, vorzugsweise C₇- bis C₁₉-Alkyl oder -Alkenyl, insbesondere C₉- bis C₁₇-Alkyl oder -Alkenyl. R¹ steht vorzugsweise für den Rest einer langkettigen Carbonsäure, insbesondere einer natürlich vorkommenden Fettsäure wie Laurinsäure, Myristinsäure, Palmitinsäure, Stearinsäure oder Ölsäure oder einer synthetisch nach der Oxo- oder der Ziegler-Methode hergestellten Carbonsäure. Es können auch Gemische verschiedener Reste R¹ auftreten.

Der Rest R² bedeutet C₁- bis C₄-Alkyl wie Methyl, Ethyl, n-Propyl, iso-Propyl, n-Butyl, iso-Butyl, sec.-Butyl oder tert.-Butyl, bevorzugt werden hiervon Methyl, Ethyl und n-Butyl.

Die Variable A steht insbesondere für 1,2-Alkylengruppen, hierbei bezeichnet A vor allem 1,2-Ethylen, daneben aber auch 1,2-Propylen, 1,2-Butylen oder 2,3-Butylen.

Der Alkoxylierungsgrad x hat einen Wert von 2 bis 6, vorzugsweise von 2,2 bis 4,5, insbesondere von 2,5 bis 4, wobei x einen statistischen Durchschnittswert, d.h. das Häufigkeitsmaximum einer Verteilungskurve, darstellen kann.

In einer besonders bevorzugten Ausführungsform setzt man endgruppenverschlossene Fettsäureamidalkoxylate I ein, bei denen
- R¹: einen C₇- bis C₁₉-Alkyl- oder -Alkenylrest bezeichnet,
- R²: Methyl, Ethyl oder n-Butyl bedeutet,
- A: für 1,2-Ethylen steht,
- n: die Zahl 2 oder 3 bezeichnet und
- x: einen Wert von 2,2 bis 4,5 hat.

Die Verbindungen I sind geeignet als nichtionische Tenside in festen oder flüssigen Reinigungsmitteln für Industrie, Gaststättengewerbe und Haushalt, insbesondere zur Reinigung harter Oberflächen, beispielsweise aus Glas, Porzellan, Keramik oder Metall, so in Handgeschirrspülmitteln. Weiterhin eignen sich die Verbindungen I auch gut für maschinell ablaufende Reinigungsprozesse in der Metall-, Papier-, Textil- oder Nahrungsmittelindustrie, beispielsweise für die industrielle Flaschenwäsche oder für die maschinelle Geschirreinigung.

Für die Reinigung von Flaschen in der Getränkeindustrie werden hochalkalische Reiniger eingesetzt. Das Alkali löst, neutralisiert bzw. verseift Getränkereste und Rückstände und führt den Etikettenleim in eine stark schäumende wasserlösliche Form über. All diese Prozesse laufen bei hoher Mechanik ab und begünstigen somit die ohnehin große Schaumneigung von Stärke und Zuckerabbauprodukten.

Eine andere Anwendung betrifft die industriellen Reinigungsprozesse in der Metallindustrie. Auch hier wird mit hohem Druck eine sehr gut netzende alkalische wäßrige Lösung als Reinigungsmedium zum Entfernen von Zieh- und Walzfetten bzw. carboxylgruppenhaltigen organischen Korrosionsinhibitoren eingesetzt. Hier sollen die erfindungsgemäß verwendeten Tenside nicht nur die Netzeigenschaften verbessern, sondern insbesondere zur Schaumdämpfung von z.B. anionischen Tensiden vom Typ der Alkylbenzolsulfonate bzw. anderer Sulfonsäuregruppen- und Carboxylgruppen-haltiger Tenside beitragen.

Die Verbindungen I eignen sich ebenfalls in hervorragender Weise als nichtionische Tenside in festen oder flüssigen Textilwaschmittelzusammensetzungen. Hierbei können in Kombination mit üblichen anionischen und/oder nichtionischen Tensiden vorteilhafte synergistische Effekte erzielt werden.

Die Verbindungen I können weiterhin mit gutem Erfolg in der Kosmetik als Emulgatoren in Körperpflegemitteln wie Hautcremes, Lotionen, Gelen, Hautölen oder Haarschampoos eingesetzt werden.

Die Verbindungen I eignen sich generell als oberflächenaktive Substanzen für industrielle Anwendungszwecke und weisen somit eine Vielzahl von weiteren technischen Anwendungsmöglichkeiten auf. Mögliche Einsatzgebiete sind hier die Galvanotechnik, die photographische Industrie, die Erdölförderung, die pharmazeutische Industrie, die Pflanzenernährung und der Pflanzenschutz; die Verbindung I eignen sich allgemein für Wirkstoffzubereitungen aus hydrophoben und hydrophilen Komponenten.

Auch bei der Herstellung von Emulsionen, Pigment- und Kunststoffdispersionen können die Verbindungen I mit gutem Erfolg als Emulgatoren oder Stabilisatoren eingesetzt werden.

Weiterhin sind Gegenstand der vorliegenden Erfindung Wasch-, Reinigungs- und Körperpflegemittel, die neben den hierfür üblichen Bestandteilen 0,1 bis 50 Gew.-%, vorzugsweise 1 bis 30 Gew.-%, bezogen auf die Gesamtmenge der Zubereitung, einer oder mehrerer Verbindungen I enthalten. Die Bestandteile und die Zusammensetzung derartiger Wasch-, Reinigungs- und Körperpflegemittel sind dem Fachmann bekannt und brauchen deshalb hier nicht näher erläutert zu werden.

Die endgruppenverschlossenen Fettsäureamidalkoxylate I sind verseifungsstabil in alkalischem und in schwach saurem Medium und zeichnen sich durch ihre guten Anwendungseigenschaften wie effiziente Erniedrigung der Oberflächenspannung, gutes Netzvermögen, gutes Schaumvermögen bei einem Teil der Verbindungen I und Schaumarmut und gute Schaumdämpfung bei einem anderen Teil der Verbindungen I, gute Hartwasserbeständigkeit sowie gutes Waschvermögen bei gleichzeitiger biologischer Abbaubarkeit und toxikologischer Unbedenklichkeit aus.

Gegenstand der vorliegenden Erfindung sind auch endgruppenverschlossene Fettsäureamidalkoxylate der allgemeinen Formel Ia

R¹-CO-NH-(CH₂)ₙ-O-(AO)_{y}-R² (Ia)

in der
- R¹: einen C₅- bis C₂₁-Alkyl- oder -Alkenylrest bezeichnet,
- R²: eine C₁- bis C₄-Alkylgruppe bedeutet,
- A: für C₂- bis C₄-Alkylen steht,
- n: die Zahl 2 oder 3 bezeichnet und
- y: einen Wert von größer 2 bis 6, vorzugsweise von 2,2 bis 4,5, insbesondere von 2,5 bis 4, hat.

Die Verbindungen Ia und auch die Verbindungen I lassen sich in vorteilhafter Weise dadurch herstellen, daß man Fettsäureester der allgemeinen Formel II

R¹-CO-O-R³ (II)

in der R³ für C₁- bis C₈-Alkyl, insbesondere C₁- bis C₄-Alkyl, steht und R¹ die oben genannte Bedeutung hat, mit Polyoxyalkylenaminen der allgemeinen Formel III

H₂N-(CH₂)ₙ-O-(AO)_{y}-R² (III)

in der R², A, n und y die oben genannte Bedeutung haben, umsetzt. Dabei werden meist basische Katalysatoren, vorzugsweise Alkalimetallalkoholate, mit eingesetzt.

Die Verbindungen Ia lassen sich auch in vorteilhafter Weise dadurch herstellen, daß man Fettsäuren der allgemeinen Formel IV

R¹-COOH (IV)

in der R¹ die oben genannte Bedeutung hat, mit den Polyoxyalkylenaminen III umsetzt. Dabei werden meist saure Katalysatoren, vorzugsweise Mineralsäuren wie Salzsäure, Schwefelsäure oder Phosphorsäure, Carbonsäuren wie Ameisensäure oder Essigsäure oder Sulfonsäuren, die auch Tensideigenschaften aufweisen können, wie p-Toluolsulfonsäure oder Dodecylbenzolsulfonsäure, eingesetzt.

Zur Herstellung der entsprechenden Verbindungen I setzt man mit Polyoxyalkylenaminen der allgemeinen Formel

H₂N-(CH₂)ₙ-O-(AO)ₓ-R²

um.

### Herstellungsbeispiele

### Beispiel 1

In einem Glasreaktor wurden 102,5 g (0,5 mol) n-Butyltriglykolamin der Formel

H₂N-(CH₂CH₂O)₃-C₄H₉

vorgelegt und auf 100°C erwärmt. Unter Rühren wurden innerhalb von 20 Minuten 110 g (0,5 mol) Dodecansäuremethylester zugetropft. Man heizte die Reaktionsmischung auf 120°C auf und tropfte bei dieser Temperatur langsam 13,5 g (entsprechend 0,075 mol NaOCH₃) einer Natriummethanolat-Lösung (30 gew.-%ig in Methanol) zu und ließ 15 Minuten bei 120°C rühren. Anschließend wurde durch Anlegen eines Vakuums der Druck langsam auf 80-100 mbar gesenkt. Nach 3 Stunden Rühren bei 120°C und 80-100 mbar wurden weitere 4,5 g der Natriummethanolat-Lösung zugegeben und die Reaktionsmischung wurde noch zwei Stunden bei 120°C und 80-100 mbar nachgerührt. Die Vollständigkeit des Umsatzes wurde durch Infrarot-Spektroskopie überprüft.

Man erhielt als Austrag 201 g eines hellbraunen, bei Raumtemperatur wachsähnlichen Feststoffes.

### Beispiel 2

Die Umsetzung wurde in Analogie zu Beispiel 1 ausgehend von 103,5 g (0,5 mol) Methyltetraglykolamin der Formel

H₂N-(CH₂CH₂O)₄-CH₃

und 110 g (0,5 mol) Dodecansäuremethylester durchgeführt.

Man erhielt als Austrag 198 g eines hellbraunen, bei Raumtemperatur wachsähnlichen Produktes.

### Beispiel 3

Die Umsetzung wurde in Analogie zu Beispiel 1 ausgehend von 110,5 g (0,5 mol) Ethyltetraglykolamin der Formel

H₂N-(CH₂CH₂O)₄-C₂H₅

und 110 g (0,5 mol) Dodecansäuremethylester durchgeführt.

Man erhielt als Austrag 200 g eines hellbraunen, bei Raumtemperatur wachsähnlichen Produktes.

### Beispiel 4

Die Umsetzung wurde in Analogie zu Beispiel 1 ausgehend von 81,5 g (0,5 mol) Methyltriglykolamin der Formel

H₂N-(CH₂CH₂O)₃-CH₃

und 110 g (0,5 mol) Dodecansäuremethylester durchgeführt.

Man erhielt als Austrag 175 g eines hellbraunen, bei Raumtemperatur wachsähnlichen Produktes.

### Beispiel 5

Die Umsetzung wurde in Analogie zu Beispiel 1 ausgehend von 88,5 g (0,5 mol) Ethyltriglykolamin der Formel

H₂N-(CH₂CH₂O)₃-C₂H₅

und 110 g (0,5 mol) Dodecansäuremethylester durchgeführt.

Man erhielt als Austrag 184 g eines hellbraunen, bei Raumtemperatur wachsähnlichen Produktes.

### Beispiel 6

Die Umsetzung wurde in Analogie zu Beispiel 1 ausgehend von 110,9 g (0,5 mol) Ethyltetraglykolamin der Formel

H₂N-(CH₂CH₂O)₄-C₂H₅

und 114,0 g (0,5 mol) eines handelsüblichen Gemisches von gesättigten C₈- bis C₁₈-Fettsäuremethylestern durchgeführt.

Man erhielt als Austrag 189 g eines hellbraunen, bei Raumtemperatur wachsähnlichen Produktes.

### Beispiel 7

Die Umsetzung wurde in Analogie zu Beispiel 1 ausgehend von 73,2 g (0,4 mol) eines Polyoxyalkylenamins der Formel

H₂N-(CH₂)₃-O-(CH₂CH₂O)₂-CH₃

und 89,8 g (0,4 mol) Dodecansäuremethylester durchgeführt.

Man erhielt als Austrag 152 g eines hellbraunen, bei Raumtemperatur wachsähnlichen Produktes.

### Beispiel 8

In einem Glasreaktor wurden 51,8 g (0,25 mol) Methyltetraglykolamin der Formel

H₂N-(CH₂CH₂O)₄-CH₃

vorgelegt und auf 100°C erwärmt. Unter Rühren wurden innerhalb von 20 Minuten 51,8 g (0,25 mol) Kokosfettsäure (Edenor® K8-18 der Fa. Henkel) zugegeben. Danach heizte man die Reaktionsmischung auf 120°C auf, fügte bei dieser Temperatur langsam 1,0 g (0,003 mol) Dodecylbenzolsulfonsäure zu und erwärmte auf 180°C. Nach 3stündigem Rühren bei 180°C destillierte man restliches Reaktionswasser bei 80 bis 100 mbar ab. Danach war durch Infrarot-Spektroskopie keine Carbonsäure mehr im Reaktionsgemisch nachweisbar. Der Reaktorinhalt wurde bei 80 bis 100°C abgelassen.

Man erhielt als Austrag 94,8 g eines bei Raumtemperatur wachsänhlichen Feststoffes.

### Anwendungsbeispiele

Der Trübungspunkt wurde gemäß DIN 53917 bestimmt.

Die Prüfung des Schäumvermögens erfolgte gemäß DIN 53902 bei 40°C mit 2 g/l Prüfsubstanz und einer Wasserhärte von 1,78 mmol Ca/l (≙ 10° dH). Dabei wurde das Schaumvolumen in ml 30 sec nach Beendigung der Schaumerzeugung bestimmt.

Zur weiteren Charakterisierung wurde das Netzvermögen durch Tauchen eines Baumwollgewebes in die zu untersuchende Tensidlösung gemäß DIN 53901 geprüft. Die Messung wurde mit 2 g/l Prüfsubstanz und 2 g/l Natriumcarbonat in destilliertem Wasser bei 20° und 70°C durchgeführt. Dabei wurde die Zeit in sec gemessen, nach der das Gewebe seinen durch die eingeschlossene Luft verursachten Auftrieb verliert und zu sinken beginnt. Je kürzer die Zeitspanne, desto besser ist das Netzvermögen.

Die Messung der Oberflächenspannung erfolgte gemäß DIN 53914 bei 20°C mit 0,1 g/l Prüfsubstanz. Dabei wurde die Kraft in mN/m gemessen, welche notwendig ist, um einen horizontal aufgehängten Ring oder Bügel aus der Flüssigkeitsoberfläche herauszuziehen.

Das Schaumdämpfungsverhalten wurde in der Geschirrspülmaschine durch den sogenannten "Ei-Test" geprüft. Hierbei wurde durch magnetische Induktionsmessung in einem handelsüblichen Haushalts-Geschirrspülautomaten mit Hilfe eines Zählwerkes die Zahl der Umdrehungen eines Sprüharmes bestimmt. Durch Schaumbildung, die besonders bei Anwesenheit von Proteinen (Eiweiß) auftritt, wird die Umdrehungszahl des Sprüharms vermindert. Die Umdrehungszahl stellt somit wegen der verringerten Rückstoßkraft ein Maß für die Tauglichkeit von Tensiden in Reinigungsgeräten mit hoher Mechanik dar. Die Testzeit betrug 12 Minuten, wobei die durchschnittliche Umdrehungszahl aus der Gesamtumdrehungszahl berechnet wurde. Der Waschvorgang wurde bei Raumtemperatur begonnen, nach etwa 10 Minuten betrug die Temperatur des Spülwassers 60°C.

Die nachfolgende Tabelle zeigt die Ergebnisse der Messungen.

| Produkt gemäß Beispiel Nr. | Trübungspunkt | Schäumvermögen | Netzvermögen | | Oberflächenspannung | "Ei-Test" |
|---|---|---|---|---|---|---|
| | | | 20°C | 70°C | | |
| | [°C] | [ml] | [sec] | [sec] | [mN/m] | [U/min] |
| 1 | 0 | 0 | 96 | 175 | 33,4 | 112 |
| 2 | 58 | 340 | 8 | 22 | 31,9 | 44 |
| 3 | 36 | 30 | 22 | 43 | 29,8 | 84 |
| 4 | 55 | 85 | 28 | 32 | 28,8 | 50 |
| 5 | 30 | 10 | 68 | 48 | 32,1 | 81 |
| 6 | 61 | 20 | 17 | 30 | 28,7 | 110 |
| 7 | 0 | 10 | 69 | 45 | 31,9 | 82 |
| zum Vergleich: | | | | | | |
| A | 100 | 280 | > 300 | > 300 | 37,6 | 20 |
| B | 99 | 140 | > 300 | 272 | 50,4 | 43 |
| C | 64 | 10 | 140 | 91 | 40,9 | 34 |

Als Vergleichssubstanz dienten nichtionische Tenside gemäß (3) der Formel

C₁₁/C₁₃-Alkyl-CO-NH-(CH₂CH₂O)₂₀-W

mit W = H (Beispiel A), CH₃ (Beispiel B) oder n-C₄H₉ (Beispiel C).

Wie den Beispielen zu entnehmen ist, zeigen die erfindungsgemäßen Substanzen deutlich günstigere Eigenschaften als die Vergleichssubstanzen.

Die Beispiele 2 und 4 zeigen Produkte, wie sie in Textilwaschmitteln oder Handgeschirrspülmitteln eingesetzt werden können. Gegenüber den Vergleichen A und B zeigen die erfindungsgemäßen Substanzen eine deutlich bessere Erniedrigung der Oberflächenspannung und ein deutlich besseres Netzvermögen.

Die Beispiele 1, 3, 5, 6 und 7 zeigen Substanzen, wie sie für technische Reiniger (maschinelle Reinigungsoperationen) geeignet sind. Neben ihrer Schaumarmut zeigen diese Substanzen eine höhere Erniedrigung der Oberflächenspannung, ein besseres Netzvermögen und besonders wichtig eine deutlich bessere Schaumdämpfung im Geschirrspülmaschinentest als beispielsweise Vergleichsubstanz C.

## Patentansprüche

1. Verwendung von endgruppenverschlossenen Fettsäureamidalkoxylaten der allgemeinen Formel I
R¹-CO-NH-(CH₂)ₙ-O-(AO)ₓ-R² (I)
in der
R¹ einen C₅- bis C₂₁-Alkyl- oder -Alkenylrest bezeichnet,
R² eine C₁- bis C₄-Alkylgruppe bedeutet,
A für C₂- bis C₄-Alkylen steht,
n die zahl 2 oder 3 bezeichnet und
x einen Wert von 2 bis 6 hat,
als nichtionische Tenside oder Emulgatoren in Wasch-, Reinigungs- oder Körperpflegemitteln, als oberflächenaktive Substanzen für industrielle Anwendungszwecke in der Galvanotechnik, in der photographischen Industrie, bei der Erdölförderung, in der pharmazeutischen Industrie, bei der Pflanzenernährung und in Pflanzenschutzformulierungen sowie als oberflächenaktive Substanzen bei der Herstellung von Emulsionen, Pigment- und Kunststoffdispersionen.

2. Verwendung von endgruppenverschlossenen Fettsäureamidalkoxylaten I nach Anspruch 1, bei denen
R¹ einen C₇- bis C₁₉-Alkyl- oder -Alkenylrest bezeichnet,
R² Methyl, Ethyl oder n-Butyl bedeutet,
A für 1,2-Ethylen steht,
n die zahl 2 oder 3 bezeichnet und
x einen Wert von 2,2 bis 4,5 hat.

3. Wasch-, Reinigungs- und Körperpflegemittel, enthaltend neben den üblichen Bestandteilen 0,1 bis 50 Gew.-% endgruppenverschlossene Fettsäureamidalkoxylate I gemäß Anspruch 1 oder 2 als nichtionische Tenside oder Emulgatoren.

4. Endgruppenverschlossene Fettsäureamidalkoxylate der allgemeinen Formel Ia
R¹-CO-NH-(CH₂)ₙ-O-(AO)_{y}-R² (Ia)
in der
R¹ einen C₅- bis C₂₁-Alkyl- oder -Alkenylrest bezeichnet,
R² eine C₁- bis C₄-Alkylgruppe bedeutet,
A für C₂- bis C₄-Alkylen steht,
n die zahl 2 oder 3 bezeichnet und
y einen Wert von größer 2 bis 6 hat.

5. Verfahren zur Herstellung von endgruppenverschlossenen Fettsäureamidalkoxylaten Ia gemäß Anspruch 4, dadurch gekennzeichnet, daß man Fettsäureester der allgemeinen Formel II
R¹-CO-O-R³ (II)
in der R³ für C₁- bis C₈-Alkyl steht und R¹ die oben genannte Bedeutung hat, mit Polyoxyalkylenaminen der allgemeinen Formel III
H₂N-(CH₂)ₙ-O-(AO)_{y}-R² (III)
in der R^{2,} A, n und y die oben genannte Bedeutung haben, umsetzt.

6. Verfahren zur Herstellung von endgruppenverschlossenen Fettsäureamidalkoxylaten Ia gemäß Anspruch 4, dadurch gekennzeichnet, daß man Fettsäuren der allgemeinen Formel IV
R¹-COOH (IV)
in der R¹ die oben genannte Bedeutung hat, mit Polyoxyalkylenaminen der allgemeinen Formel III
H₂N-(CH₂)ₙ-O-(AO)_{y}-R² (III)
in der R², A, n und y die oben genannte Bedeutung haben, umsetzt.

## Claims

1. The use of endgroup-capped fatty amide alkoxylates of the general formula I
R¹-CO-NH-(CH₂)ₙ-O-(AO)ₓ-R² (I)
where
R¹ is C₅-C₂₁-alkyl or -alkenyl,
R² is C₁-C₄-alkyl,
A is C₂-C₄-alkylene,
n is 2 or 3, and
x has a value from 2 to 6,
as nonionic surfactants or emulsifiers in detergents, cleaners or body care compositions, as surface-active substances for industrial applications in electroplating, in the photographic industry, in petroleum production, in the pharmaceutical industry, in plant feeding and in crop protection formulations, and as surface-active substances in the production of emulsions and pigment and plastic dispersions.

2. The use of endgroup-capped fatty amide alkoxylates I as claimed in claim 1, where
R¹ is C₇-C₁₉-alkyl or -alkenyl,
R² is methyl, ethyl or n-butyl,
A is 1,2-ethylene,
n is 2 or 3, and
x has a value from 2.2 to 4.5.

3. A detergent, cleaner or body care composition which, besides conventional ingredients, contains from 0.1 to 50% by weight of endgroup-capped fatty amide alkoxylates I as claimed in claim 1 or 2 as nonionic surfactants or emulsifiers.

4. An endgroup-capped fatty amide alkoxylate of the formula Ia
R¹-CO-NH-(CH₂)ₙ-O-(AO)_{y}-R² (Ia)
where
R¹ is C₅-C₂₁-alkyl or -alkenyl,
R² is C₁-C₄-alkyl,
A is C₂-C₄-alkylene,
n is 2 or 3, and
y has a value from greater than 2 to 6.

5. A process for preparing endgroup-capped fatty amide alkoxylates Ia as claimed in claim 4, which comprises reacting fatty acid esters of the general formula II
R¹-CO-O-R³ (II)
where R³ is C₁-C₈-alkyl, and R¹ has the abovementioned meaning, with polyoxyalkylene amines of the general formula III
H₂N-(CH₂)ₙ-O-(AO)_{y}-R² (III)
where R^{2,} A, n and y have the abovementioned meanings.

6. A process for preparing endgroup-capped fatty amide alkoxylates Ia as claimed in claim 4, which comprises reacting fatty acids of the general formula IV
R¹-COOH (IV)
where R¹ has the abovementioned meaning, with polyoxyalkylene amines of the general formula III
H₂N-(CH₂)ₙ-O-(AO)_{y}-R² (III)
where R², A, n and y have the abovementioned meanings.

## Revendications

1. Utilisation d'alcoxylates d'amides d'acides gras à groupements terminaux bloqués de formule générale I
R¹-CO-NH-(CH₂)ₙ-O-(AO)ₓ-R² (I)
dans laquelle
R¹ représente un reste alkyle ou alcényle en C₅-C₂₁,
R² représente un groupement alkyle en C₁-C₄,
A est mis pour un reste alkylène en C2-C4,
n représente le nombre 2 ou 3 et
x a une valeur de 2 à 6,
comme tensio-actifs ou émulsifiants non ioniques dans des agents de lavage, de nettoyage ou d'hygiène corporelle, comme substances actives pour une utilisation industrielle en galvanoplastie, dans l'industrie photographique, dans l'exploitation pétrolière, dans l'industrie pharmaceutique, pour l'alimentation des plantes et dans des formulations phytosanitaires ainsi que comme substances actives pour la préparation d'émulsions, de dispersions de pigments et de matières plastiques.

2. Utilisation des alcoxylates d'amides d'acides gras à extrémités bloquées selon la revendication 1, composés dans lesquels
R¹ représente un reste alkyle ou alcényle en C₇-C₁₉,
R² représente un groupement méthyle, éthyle ou n-butyle,
A est mis pour un groupement 1,2-éthylène,
n représente le nombre 2 ou 3 et
x a une valeur de 2,2 à 4,5.

3. Agents de lavage, de nettoyage ou d'hygiène corporelle, contenant outre les constituants usuels, de 0,1 à 50% en poids des alcoxylates d'amides d'acides gras à extrémités bloquées I selon la revendication 1 ou 2 comme tensio-actifs ou émulsionnants non ioniques.

4. Alcoxylates d'amides d'acides gras à extrémités bloquées de formule générale Ia
R¹-CO-NH-(CH₂)ₙ-O-(AO)_{y}-R² (Ia)
dans laquelle
R¹ représente un reste alkyle ou alcényle en C₅-C₂₁,
R² représente un groupement alkyle en C₁-C₄,
A est mis pour un groupement alkylène en C₂-C₄,
n représente le nombre 2 ou 3 et
y a une valeur de plus de 2 à 6.

5. Procédé de préparation d'alcoxylates d'amides d'acides gras à extrémités bloquées Ia selon la revendication 4, caractérisé en ce qu'on fait réagir des esters d'acides gras de formule générale II
R¹-CO-O-R³ (II)
dans laquelle R³ représente un groupement alkyle en C₁-C₈ et R¹ a la signification donnée ci-dessus, avec des polyoxyalkylène-amines de formule générale III
H₂N-(CH₂)ₙ-O-(AO)_{y}-R² (III)
dans laquelle R², A, n et y ont les significations données ci-dessus.

6. Procédé de préparation des alcoxylates d'amides d'acides gras à extrémités bloquées Ia selon la revendication 4, caractérisé en ce qu'on fait réagir des acides gras de formule générale IV
R¹-COOH (IV)
dans laquelle R¹ a la définition donnée ci-dessus, avec des polyoxyalkylène-amines de formule générale III
H₂N-(CH₂)ₙ-O-(AO)_{y}-R² (III)
dans laquelle R², A, n et y ont les significations données ci-dessus.
